# EUROPEAN PATENT SPECIFICATION

(11) **EP 0 806 889 B1**
(45) Date of publication and mention of the grant of the patent: **19.04.2006**
(21) Application number: 97100825.5
(22) Date of filing: 21.01.1997
(51) Int. Cl.: H05F 7/00

(54) **Environment improving implement and environment improving method**
Verfahren und Anordnung zur Verbesserung der Umwelt
Méthode et dispositif pour améliorer l'environnement

(30) Priority: 09.05.1996 JP 11510496; 26.06.1996 JP 16632796
(43) Date of publication of application: 12.11.1997
(73) Proprietor: Takamatsu, Kuniaki, Kaminoyama-shi, Yamagata 999-31 (JP); Ohara, Toyoko, Nara-shi, Nara 631 (JP); Tokumaru, Masakazu, Oita-shi, Oita 870-02 (JP)
(72) Inventor: Takamatsu, Kuniaki, Kaminoyama-shi, Yamagata 999-31 (JP); Ohara, Toyoko, Nara-shi, Nara 631 (JP); Kikuchi, Hideaki, Osaka-shi, Osaka 535 (JP)
(74) Representative: Dallmeyer, Georg

(56) References cited:
- EP-A- 0 551 668
- WO-A-93/17753
- BE-A- 693 517
- DE-A- 4 317 884
- FR-A- 2 096 665
- GB-A- 1 167 053

## Description

The invention relates to an electrification preventing implement and an method electrification preventing according to claims 1 and 2, wherein minus ions are used for improving environment by removing static electricity.

It is generally known that stray current flows in the atmosphere and the earth. The intensity varies depending on changes in atmospheric potential caused by changes in weather, geographic features, and other natural conditions, and also depending on artificial things such as electric apparatuses, railways, power cables, and so forth and is influenced with metalliferous veins, ground water arteries, and underground cables, if they are present. Ordinarily, in the ground with a height above the sea of 100 m, earth current with an intensity of about 15 *µ*A flows. However, in the modern society, with the spread of electric apparatus, the intensity of the stray current has been higher almost in any place. This appears as static electricity which is electrified on the earth. In event that earth current having an intensity of 30 to 40 *µ* A flows through a human body to electrify, there is a great possibility that the current causes various hindrances to men's health. Light symptoms caused by the electrification include stiffness in the shoulder, headache, fatigue feeling, and so forth. However, if the electrification lasts in a long term, or the charge quantity is high, the morbid symptoms become severe. In some cases, their human relations are badly influenced with the poor physical conditions.

The electrification exerts a bad influence not only over human bodies but also over plants, animals, and even objects. The bad influences can be appreciated as follows; for plants, the growing rate is decreased, the vitality of forests is deteriorated, and for animals, the vitality is reduced, and the morbid symptoms appear. In an electrified condition, cat fish can hardly foreknown an earthquake, cat fish in the river being well-known as an animal having ability to foreknow an earthquake in Japan.

Though the electrostatic charge, generated by the electrification of an object, is effectively used in some cases, they may cause the following hindrances: mechanical hindrances occur, that is, electrified objects such as powder, fibers, sheets, and so forth get to be caught in other objects, combustible and explosive objects are ignited when static electricity is discharged, which may induce fires and explosions, and the electrostatic charge may be a cause of the breaking, error action, quality-deterioration of precision apparatuses such as semiconductor parts, and so forth.

Conventionally, as a method of inhibiting the residence of earth current to prevent the electrification in a wide area the embedment of carbon is carried out. Hereinafter, the embedment of carbon will be described.

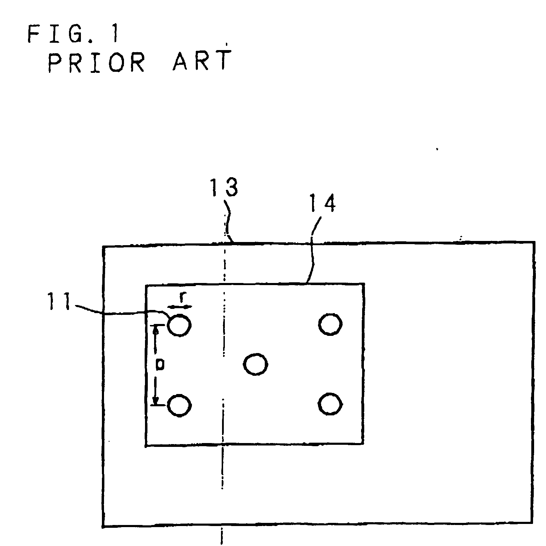

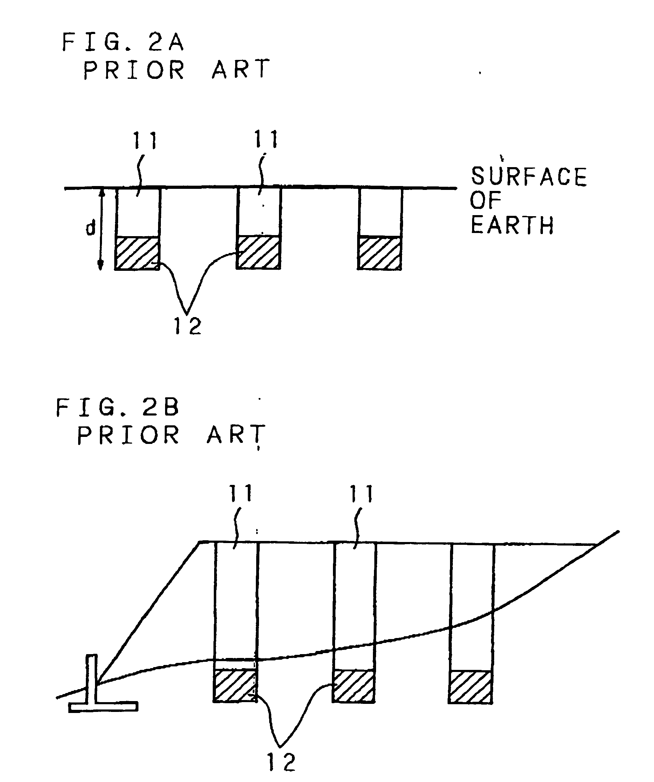

For example, as shown in FIG. 1, for a building lot wi th a land area of 100 tsubo (1 tsubo = 3.3m²) and a building area of 40 tsubo, embedment pits 11 with a diameter (r) of 1 m and a depth (d) of 1 to 2 m are dug at intervals (p) of 5 to 10 m. 200 to 500 kg per pit of carbon 12 (for example, coconut shell carbon) is embedded into a pit. The carbon 12 may have a form of grains or powder, depending on the conditions of the land 13 and the building 14. The intervals between the embedment pits 11 can be changed depending on the conditions of the land and the building. FIGS. 2A and 2B are sectional model views of the embedment pits 11. FIG. 2A shows pits provided in a level ground. The pits shown in FIG. 2B are provided in a sloping land (the building lot is an embankment). In the case of such sloping lands as shown in FIG. 2B, the carbon 12 is embedded in the mountain-shape land at the same horizontal-level.

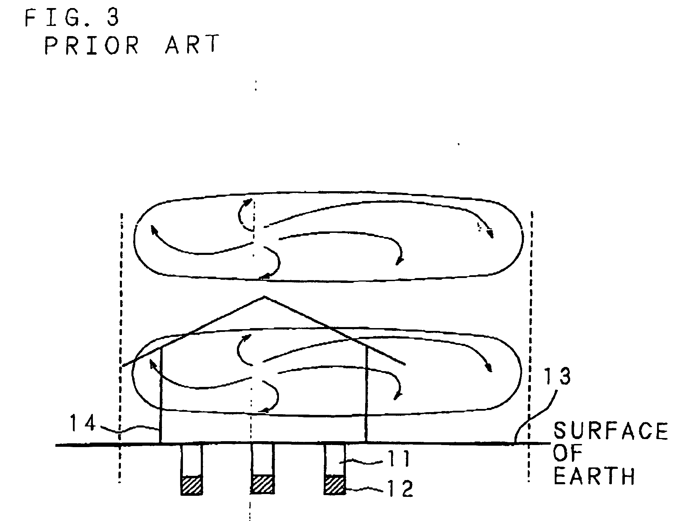

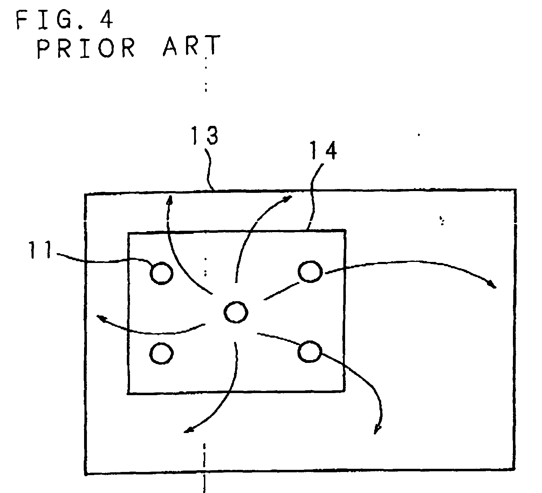

When the carbon is embedded, the electric resistance is reduced as a whole, so that the stray current flows, swirling and spreading as shown in FIG. 3 (side view) and FIG. 4 (plan view). This reduces the electrification of men, plants and animals, and objects present in the land in which the carbon is embedded, that is, reduces the static electricity on the ground. Such phenomena are effective to a height above the sea level of about 3 m, which serves for the stabilization of them.

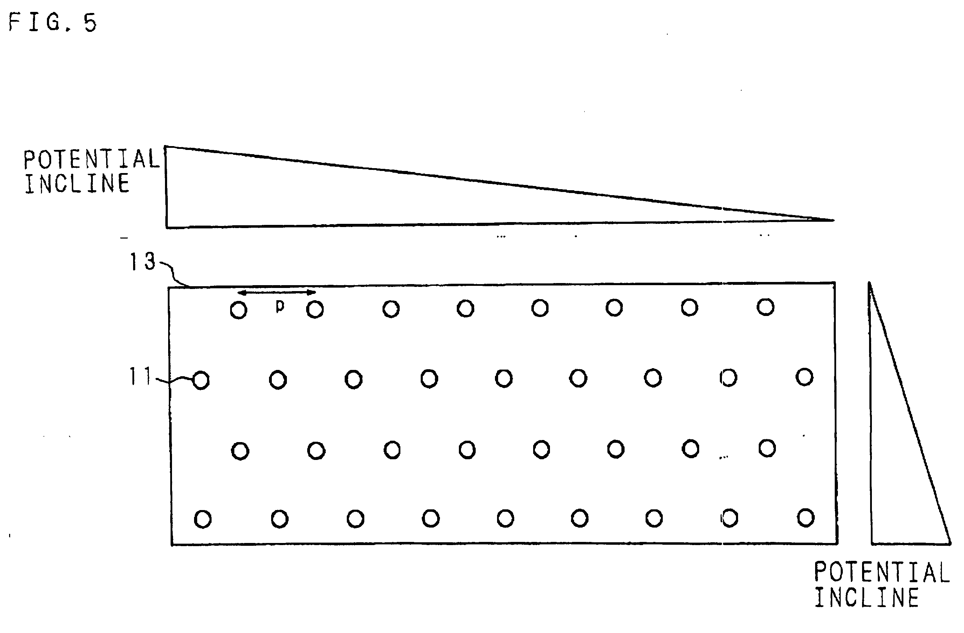

FIG. 5 is a plan view which illustrates the application to a factory and a farm. The embedment pits 11 for embedding the carbon 12 have an interval (p) of 10 m, are provided in the sites positioning at the apexes of a triangle. The embedment quantities of the carbon 12 are controlled according to the potential inclination. In the case of the potential inclination to be provided in the lateral direction as shown in FIG. 5, 200 kg of the carbon 12 is embedded in the embedment pit 11 in the highest potential site, and 500 kg of the carbon 12 is done in the embedment pit 11 in the lowest potential site. Between both sites, the quantities of the carbon to be embedded are determined according to the proportional distribution. Also, in the case of the potential inclination in the longitudinal direction of the drawing, the quantities of the carbon to be embedded are controlled. The embedment pits 11 have a diameter of 1 to 1.2 mm and a depth of 1 to 2 m. In this case, it is desirable that the carbon 12 is embedded in the mountain-shape land at the same horizontal level. The case where the carbon embedment is applied to the factory and farm can also obtain a similar effect to the case of residential land.

When the embedment of the carbon is carried out, 1 to 2 tons of the carbon 12 is required for a house. For the application to a factory (lot 1000 tsubo, building 500 tsubo), 10 to 30 tons of the carbon 12 is needed. In addition, many embedment pits 11 are dug. The overall amount of money including the construction cost is vast. The economical burden is very heavy.

When carbon is sprayed on the ground surface to a thickness of about 3 to 10 cm, effects to some degree can be obtained. However, the effects are lower as compared with the underground embedment, and the effectiveness-lasting time is short, that is, about half a year.

The influence of static electricity is more apparently recognized indoor, as compared with the outdoor condition. For the purpose of making indoor air more comfortable or activating the indoor air, air cleaning devices having function for generating minus ions, infrared ray generators, static-electricity removing devices, dehumidifiers, humidifiers, and so forth have been rapidly spread. In addition, bedding (futon, pillows), clothes, cosmetics, and so forth which allow minus ions to act on human bodies have been practically used.

The effects of minus ions can be explained based on the biological action of atmospheric ions or the principle of the ion introduction.

The causes of the atmospheric ions include ionization effects by space rays, ultraviolet rays, natural radioactive substances, atmospheric discharge (lightning), and so forth, and triboelectricity generated at snow falling, effects accompanied by the changes in surface area of water drops, the Lennard effect, and so forth. Also, the phenomenon is called global circuit wherein minus ions flow between ionosphere and earth circularly, and current flows in the opposite direction. It is generally thought that as the biological actions of the atmospheric ions generated by the above-mentioned causes, the minus ion alkaline atmosphere has systematic actions such as tranquil, invigorating, hypnotic, appetite promoting actions, and so forth, blood pressure drop action, blood sugar reduction, vasodilatation, diuretic action, and so forth. On the other hand, it is generally thought that the plus ion acidic atmosphere has systematic actions such as excitable, unpleasant feeling sleeplessness, appetite diminishment, and so forth, blood pressure rising action, blood sugar increasing action, vasoconstriction, diuretic inhibition action, and so forth.

The principle of the ion introduction is that various substances are ionized by the application of electrolytic action, and through the ions an objective substance itself is permeated into subcutaneous tissues. It is generally thought that when the activated oxygen is absorbed into a layer near the skin surface, the acidic horny layer is neutralized to be softened and the metabolism of the skin becomes active, recovering the skin function to its normal state.

In addition, the minus ions enhance the blood flow in the skin, adjusts the function of the autonomic nervous system, makes the blood alkaline so as to activate the functions of the cells. Thus, the minus ions has a positive action to prevent the skin aging and dissolve other troubles. Moreover, the minus ions, normalizing the skin functions, are very effective in prevention of the loss of hair and hair restoration.

In the indoor condition, minus ions, when they are generated with a device having a minus ion generating function, neutralizes the plus charge and further can convert to the minus ion environment. However, when the removal of electrostatic charge or the activation of a space is attempted with an electric apparatus, there exists such a problem that the electrostatic charge quantity is increased, due to the fact that the electric apparatus itself is charged, even though the air is cleaned.

Moreover, it is desired to clean water and air which are indispensable to the preservation of our life.

Conventionally, drink water is obtained by treating river water and groundwater (raw water) to clean, if necessary. As means for treating water to clean, (1) the flocculation of suspended matters, and the solid - liquid separation, (2) the coagulation of dissolved matters, oxidation, biochemical modification, adsorption, and ion exchange, (3) the agglutination and filtration of bacteria and virus, and their disinfection, (4) the neutralization of acids and alkali, and so forth are exemplified, and are employed in combination of them depending on the uses.

In general, underground water, not containing objects to be removed, has good water qualities. Therefore, the underground water, after it is subjected to chlorine disinfection only, is supplied. On the other hand, for river water and lake water, viscosity colloids, algae plankton, and contaminants deriving from the nature are objects of the water cleaning. For this purpose, the slow sand filtration or quick sand filtration system is employed.

According to the slow sand filtration system, in addition to the mechanical filtration of suspended matters, the oxidation, adsorption and decomposition of ammonia, iron, manganese, organic components, and smell components with microorganisms propagating in the surface layer, the predation with water bacteria, and the stabilization of nitrogen components with bacteria in the sand layer can be achieved.

According to the quick sand filtration system, viscosity colloids and color colloids in water are flocculated by the addition of a coagulant having a positive charge, and then the flocculation is precipitated and filtrated to be separated. As the coagulant, aluminium salts are mainly used. In part, iron salts and high polymeric substances are used. Even by the quick sand filtration system, dissolved contaminants can be flocculated, precipitated and filtrated to be cleaned to some degree, using an oxidizing agent, the absorption into aluminium hydroxide, and the coagulation. For extreme contamination and bad smells, the oxidization with ozone and the adsorption to active carbon are further used in some cases.

Water with physiological safety for drinking or tasteful water contains minerals dissolved out of rocks and stones in an appropriate amount. In the case of ultra-pure water required, processes such as ultra fine filtration, multi-step reduced-pressure distillation, ion exchange, reverse osmosis, and so forth are used to produce pure water on a required water-quality level. As described above, many water cleaning methods suitable for the respective uses of water have been developed,

In recent years, with the more contamination of raw water, the amounts of chlorine and chlorinated lime used in the above-described processes have been increased. However, there have been more people who are sensitive to the taste and smell of the chlorine and the chlorinated lime and dislike them. For this reason, water cleaning devices for cleaning tap water in their homes have been widely spread. There are various types of such water cleaning devices. That is, ones are attached to taps, and others are placed in the cabinets of sinks, and so forth. However, the water cleaning devices to be attached to taps have such problems that the taps become heavy to be handled, persons using tap water can not sufficiently see the working space under the water cleaning devices. For the water cleaning devices to be placed in the cabinets of sinks, there are such problems that the spaces for working in the cabinets are reduced. For both types of the water cleaning devices, it is necessary to maintain them, e. g., exchange the fillers.

For preventing of indoor bad smells, deodorants using active carbon and so forth, deodorizing sprays, perfumes for emitting various scents, and so forth are available. With conventionally used deodorants and deodorizing sprays, it is difficult to deodorize smells sufficiently. As to perfumes, perfumes are liked or disliked by different persons. In some cases, persons feel that perfumes smell bad.

In addition, air conditioners having an air cleaning function have been spread. However, their spread is inhibited because the devices are too expensive, the large-scale attachment is necessary, and for use of the devices, electricity is required.

DE 43 17 884 A discloses a screening device using a non-metallic container in which a mineral is received. The mineral may comprise rose quartz in granular form and the container is made of plastics or an epoxy resin.

BE 693 517A discloses a device for modifying the static electricity and its effects on the human body and more particularly on the body of a passenger of an automotive vehicle. The device comprises a case provided with suspension means, whereby it can be located at a selected point on the wearers body. The case consists of an electrically conductive material having one or more perforated faces and containing at least one water-soluble salt of an electropositive metal.

EP 0 551 668 Al discloses a resonator consisting of a mixture of rare earth compounds contained inside a plastic sphere of small diameter.

The present invention has been devised to solve abovementioned problems. A main object of the invention is to provide an electrification preventing implement and an electrification preventing method with use of minus ions.

This object is solved by providing an electrification preventing device and a method of preventing electrification according to claims 1 and 2 which are applicable to reduce electrification of static electricity due to stray current easily, using relative inexpensive means, and prevent a human body, a plant, an animal, and an object from being electrified.

An electrification preventing device of the invention is characterized in that a predetermined granular and/or powdery element and/or compound is sealed in a glass tube. Said element and/or compound may be Si and/or SiOₓ (0<x<2).

A method of preventing electrification of the invention is characterized in that the electrification preventing device is embedded underground in the vertical direction.

It is preferred to use an element or compound minus ionized previously. Higher effects can be obtained as the ionization degree is higher.

The above and further objects and features of the invention will more fully be apparent from the following detailed description with accompanying drawings.

### BRIEF DESCRIPTION OF THE SEVERAL VIEWS OF THE DRAWINGS

FIG. 1 is a plan view showing a case of an application of carbon embedment to a residential land;
FIG. 2A is a schematic sectional view showing embedment pits provided in a level ground;
FIG. 2B is a schematic sectional view showing embedment pits provided in a sloping land;
FIG. 3 is a side view explaining air discharge of a stray current;
FIG. 4 is a plan view explaining air discharge of the stray current;
FIG. 5 is a plan view explaining an application of carbon embedment to a factory or a farm;
FIG. 6 is a schematic view showing an electrification preventing implement of the invention;
FIG. 7 is a side view of a house in which the electrification preventing implement shown in FIG. 6 is embedded and its neighborhood;
FIG. 8 is a plan view of the house and its neighborhood;

Hereinafter, the present invention will be described in detail, in reference to the drawings showing embodiments.

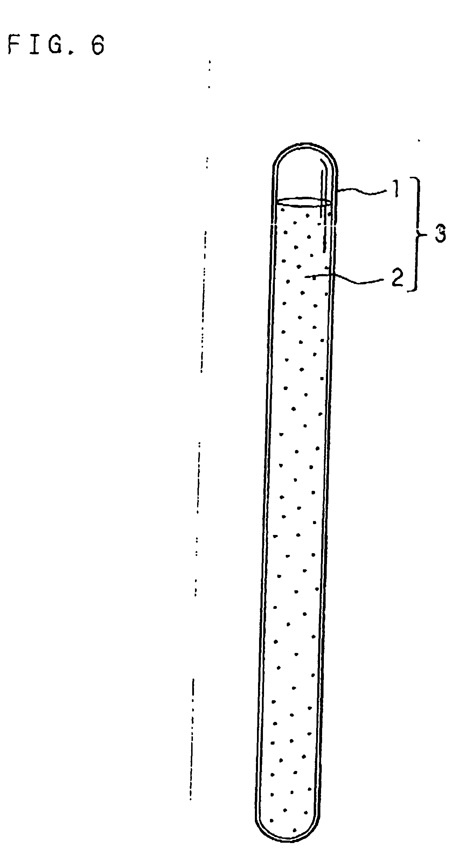

FIG. 6 is a schematic view showing an electrification preventing implement of the present invention. In a Pyrex tube (reinforced glass tube) 1, 500 to 600 g of granule or powder of, for example, silicon (Si) 2 is tightly sealed. The silicon 2 is previously placed in a minus-ionized quartz crucible for a predetermined time to be minus-ionized.

An appropriate hole is dug nearly in the center of a house, a factory, or a farm. One electrification preventing implement 3 is vertically embedded in the hole.

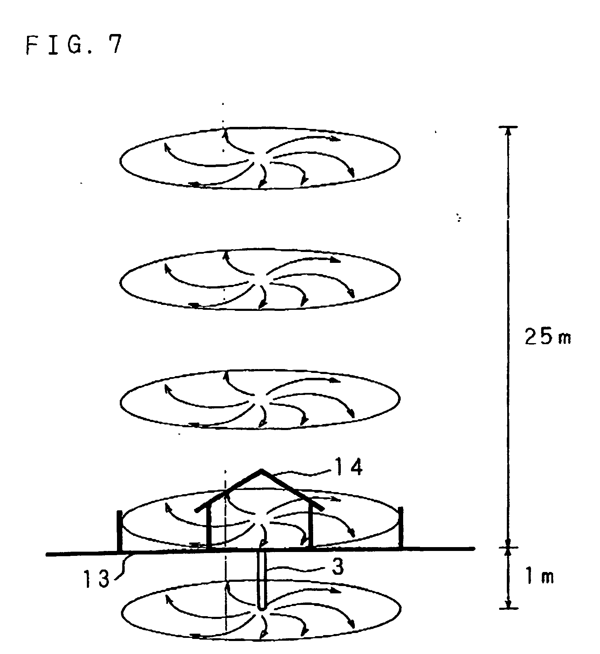

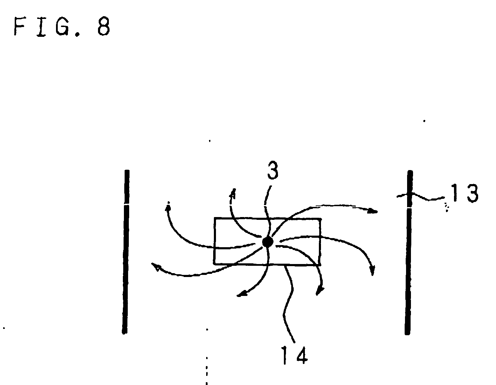

FIG. 7 is a side view of the house in which the electrification preventing implement 3 is embedded and its neighborhood. FIG. 8 is a plan view of the house and its neighborhood. When flow current is measured using a tester to quantify static electricity in the land 100 m above the sea level, the result was 30 to 40 *µ*A. The static electricity was reduced to about 15 *µ*A by the embedment of the electrification preventing implement 3. Probably, this is because the stray current on the ground surface and its neighborhood was allowed to flow easily and the residing static electricity was discharged to be eliminated. The embedment of the implement 3 reduces the intensity of current flowing human body, inhibiting the above-mentioned hindrances to men's healthy and morbid symptoms.

With the clockwise eddy of electric current as shown in FIGS. 7 and 8, minus ions from the silicon 2 so act that the concentration of minus ions in the house and its neighborhood is increased. It is generally known that the minus ions are effective in recovery from fatigue, cleaning of blood, and so forth. Air cleaning implements, bedding (futon, pillows), and so forth having such functions have been widely used. With the electrification preventing implement 3 embedded, minus ions from the silicon 2 activates hydrogen molecules. Thus, a comfortable living space in which the air is cleaned can be obtained. As to human bodies, effects whether they are physical or mental can be appreciated. For example, a person feels himself agile, becomes more patient, and so forth.

According to the method of this invention, a hole for embedding only one electrification preventing implement 3 is merely dug. The method can be carried out more easily as compared with conventional embedment of carbon. As only one glass tube is embedded, the implement can be easily transported.

In a fish feed factory filled with bad smells, the bad smells disappeared immediately after the electrification preventing implement 3 was embedded.

When the implement 3 was applied to a farm, plants were activated, and the growth rate was enhanced.

When the three to six implements 3 are embedded in a lake, the water is cleaned.

It is verified that the effects of the electrification preventing implement 3, which has a length of 1 m, extends to the space about 25 m above the ground level. In the case of a length of 1.5 m, the effects extend to the space about 50 m above the ground level. Thus, the length and the diameter of the Pyrex tube 1, and the amount of the silicon 2 can be appropriately selected, depending on lands and buildings, The effects are long-lasting. A plurality of implements 3 may be embedded. The larger number of the implements 3 is, the larger effect is obtained.

Though Si is used in the above embodiments, SiOₓ may be used. With component of SiOₓ, good effects can be obtained provided that x is a number of from 1.00 to 1.95.

Higher effects can be obtained as the ionization degree is higher.

As abovementioned, the embedment of a glass bar including silicon therein allows stray current to flow easily under and above ground. This can prevent electrification having a bad influence on a human body, animals, plants and objects.

## Claims

1. An electrification preventing implement (3) wherein a granular element (2) is sealed in a container,
**characterized in that**
said container is a tubular glass container (1), that a granular and/or powdery element or compound (2) consisting of Si or SiOₓ (0<x<2) is sealed in said tubular glass container (1) and that said element or compound (2) being minus-ionized.

2. An electrification preventing method wherein the electric preventing implement (3) of claim 1 is embedded underground in the earth in a vertical direction.

## Patentansprüche

1. Elektrifizierungs-Verhinderungsvorrichtung (3), bei der ein körniges Element (2) in einem Behälter versiegelt ist,
**dadurch gekennzeichnet, dass**
der Behälter ein rohrförmiger Glasbehälter (1) ist, ein körniges und/ oder pulvriges Element oder Gemisch (2) aus Si oder SiOₓ (0<x<2) in dem rohrförmigen Glasbehälter (1) versiegelt ist und das Element oder Gemisch (2) minus-ionisiert ist.

2. Elektrifizierungs-Verhinderungsverfahren, bei dem die Elektrifizierungs-Verhinderungsvorrichtung (3) nach Anspruch 1 in vertikaler Richtung unterirdisch in der Erde eingebettet ist.

## Revendications

1. Instrument (3) empêchant l'électrisation, dans lequel un élément granulaire (2) est enfermé hermétiquement dans un conteneur, **caractérisé en ce que** ledit conteneur est un conteneur tubulaire en verre (1), un élément ou composé (2) granulaire et/ou pulvérulent constitué de Si ou de SiOₓ (0 < x < 2) étant enfermé hermétiquement dans ledit conteneur tubulaire en verre (1) et ledit élément ou composé (2) étant ionisé à une polarité négative.

2. Procédé empêchant l'électrisation, dans lequel l'instrument (3) empêchant l'électrisation selon la revendication 1 est enfoui dans la terre dans une direction verticale.
